## Europäisches Patentamt
## European Patent Office
## Office européen des brevets

(19)

(11) Veröffentlichungsnummer: **0 377 739**

**A1**

(12) **EUROPÄISCHE PATENTANMELDUNG**
**veröffentlicht nach Art. 158 Abs. 3 EPÜ**

(21) Anmeldenummer: 88906825.0

(22) Anmeldetag: 25.05.88

(86) Internationale Anmeldenummer:
PCT/SU88/00118

(87) Internationale Veröffentlichungsnummer:
WO 89/11474 (30.11.89 89/28)

(51) Int. Cl.5: **C07D 213/53**, //A61K31/44

(43) Veröffentlichungstag der Anmeldung:
**18.07.90 Patentblatt 90/29**

(84) Benannte Vertragsstaaten:
**AT BE CH DE FR GB IT LI NL SE**

(71) Anmelder: **KIEVSKY NAUCHNO-ISSLEDOVATELSKY INSTITUT FARMAKOLOGII I TOKSIKOLOGII**
**ul. Ezhena Potie, 14**
**Kiev, 252057(SU)**

(72) Erfinder: **PANTELEIMONOV, Alexandr Gavrilovich**
**ul. Khorolskaya, 10-10**
**Kiev, 252090(SU)**
Erfinder: **LOBODA, Julia Ivanovna**
**ul. Vladimiro-Lybedskaya, 22-37**
**Kiev, 252150(SU)**
Erfinder: **LITVINOV, Valentin Borisovich**
**ul. Erevanskaya, 13-1-94**
**Kiev, 252087(SU)**
Erfinder: **MUKHINA, Luiza Vladimirovna**

**ul. Sportivnaya, 10-18**
**Kiev, 252067(SU)**
Erfinder: **TRINUS, Fedor Petrovich**
**pr. Pobedy, 12-13**
**Kiev, 252058(SU)**
Erfinder: **PORTNYAGINA, Vera Alexandrovna**
**ul. Bozhenko, 13**
**Kievskaya obl. Boyarka, 255510(SU)**
Erfinder: **FADEICHEVA, Alexandra Georgievna**
**ul. Karla Marxa, 5-11**
**Kiev, 252002(SU)**
Erfinder: **MIRIAN, Natalya Ivanovna**
**ul. Chelyabinskaya, 3-125**
**Kiev, 252097(SU)**
Erfinder: **KULIK, Ljudmila Sergeevna**
**ul. Baumana, 7/2-69**
**Kiev, 252190(SU)**

(74) Vertreter: **von Füner, Alexander, Dr.**
**Patentanwälte v. Füner, Ebbinghaus, Finck**
**Mariahilfplatz 2 & 3**
**D-8000 München 90(DE)**

(54) **VERFAHREN ZUR HERSTELLUNG VON N-ALLYL-2-PYRIDINALDOXIMBROMID.**

(57) Die vorliegende Erfindung bezieht sich auf die organische Chemie.

Das Verfahren zur Herstellung von N-Allyl-2-pyridinaldoximbromid besteht darin, dass α-Pyridinaldoxim mit Allylbromid in Medium von Dimethylsulfoxyd oder Dimethylformamid bei einer Temperatur von 40 bis 60 °C mit darauffolgender Isolierung des Zielprodukts umgesetzt wird.

Das erhaltene N-Allyl-2-pyridinaldoximbromid wird in der Medizin als Wirkstoff eines Antidots bei der Vergiftung mit phosphoroganischen Verbindungen verwendet.

VERFAHREN ZUR HERSTELLUNG VON
N-ALLYL-2-PYRIDINALDOXIMBROMID

Gebiet der Technik

Die vorliegende Erfindung bezieht sich auf die organische Chemie und betrifft insbesondere ein Verfahren zur Herstellung von N-Allyl-2-pyridinaldoximbromid, das in der Medizin als Wirkstoff eines Antidots bei der Vergiftung mit phosphoroorganischen Verbindungen Verwendung findet.

Zugrundeliegender Stand der Technik

Es ist ein Verfahren zur Herstellung von N-Allyl-2-pyridinaldoximbromid bekannt, dass darin besteht, dass 2-Pyridinaldoxim mit Allylbromid im Medium des Äthylalkohols bei der Siedetemperatur der Reaktionsmasse (70 bis 80°C) umgesetzt wird. Die Ausbeute am Zielprodukt beträgt 29 Ma-% (E.J.Poziemek, B.E.Hackley, IR, G.M.Steinberg, J. Organ, Chem. N° 5, 1958, p. 714--717).

Das genannte Verfahren wird durch eine niedrige Ausbeute und Qualität des Zielproduktes gekennzeichnet. Der Schm.p. des Zielproduktes von 300°C zeugt vom Vorliegen von Beimischungen im Zielprodukt.

Offenbarung der Erfindung

Der Erfindung liegt die Aufgabe zugrunde, durch die Änderung technologischer Vorgänge die Ausbeute am Zielprodukt zu erhöhen und das Zielprodukt von hohem Reinheitsgrad zu erhalten, das in der medizinischen Praxis verwendet werden darf.

Die Aufgabe wird dadurch gelöst, dass in einem Verfahren zur Herstellung von N-Allyl-2-pyridinaldoximbromid durch die Umsetzung von $\alpha$-Pyridinaldoxim mit Allylbromid im Medium eines organischen Lösungsmittels mit darauffolgender Isolierung des Zielproduktes erfindungsgemäss als organisches Lösungsmittel Dimethylformamid oder Dimethylsulfoxyd ausgenutzt wird und der Prozess bei einer Temperatur von 40 bis 60°C durchgeführt wird.

Das erfindungsgemässe Verfahren gestattet es, die Ausbeute am Zielprodukt bis 80 Ma-% zu vergrössern und den Reinheitsgrad, des Zielproduktes bis 100% zu erhöhen (Schm.p. beträgt 185 bis 188°C).

Der Prozess kann unter Erwärmen auf eine Temperatur von 40 bis 60°C verweirklicht werden. Zur Erhöhung der Ausbeute am Zielprodukt und Beschleunigung des Prozesses ist es zweckmässig, diesen bei einer Temperatur von 50 bis 60°C durchzuführen.

Die beste Durchführungsform der Erfindung

Das erfindungsgemässe Verfahren wird folgenderweise verwirklicht. 2-Pyridinaldoxim wird mit Allylbromid im Medium von Dimehhylformamid oder Dimethylsulfoxyd umgesetzt. Der Prozess wird bei einer Temperatur von 40 bis 60°C durchgeführt. Es ist zweckmässig, den Prozess bei einer Temperatur von 50 bis 60°C durchzuführen. Das erhaltene Zielprodukt wird abfiltriert, mit einem organischen Lösungsmittel durchgewaschen. Die Ausbeute am Zielprodukt beträgt bis 80 Ma-%, der Reinheitsgrad des Zielproduktes beträgt 100%.

Die Durchführung des Prozesses bei einer Temperatur über 60°C führt zur Verharzung des Zielproduktes und zu einer starken Verminderung dessen Ausbeute.

Die Durchführung des Prozesses bei einer Temperatur unter 50°C verlängert die Reaktionsdauer. Die Ausbeute am Zielprodukt sinkt dabei.

Das erfindungsgemässe Verfahren gestattet es, die Ausbeute am Zielprodukt (auf 80 Ma-%) im Vergleich zum bekannten Verfahren (29 Ma-%) zu erhöhen sowie das Zielprodukt von einem hohen Reinheitsgrad zu erhalten, der für dessen Anwendung in der medizinischen Praxis notwendig ist.

Zum besseren Verständnis der vorliegenden Erfindung werden folgende Beispiele der Verwirklichung des erfindungsgemässen Verfahrens angeführt.

Beispiel 1.

0,05 Mol $\alpha$-Pyridinaldoxim werden in 20 ml Dimethylsulfoxyd gelöst, es werden 0,15 Mol Allylbromid zuge-setzt und bei einer Temperatur von 60°C innerhalb von 2,5 Stunden unter Rühren erwärmt. Das erhaltenen Produkt wird abfiltriert, mit Azeton durchgewaschen. Die Aus-beute des Zielprodukts - N-Allyl-2-pyridinaldoximbromid beträgt 80 Ma-%. Schm.p. = 185 bis 188°C (aus Äthanol).

Gefunden, %: Br 32,74; 32,96; N 11,69. $C_9H_{11}BrN_2O$.

Berechnet, %: Br 32,87; N 11,52.

Beispiel 2.

0,05 Mol $\alpha$-Pyridinaldoxim werden in 200 ml Dimethylformamid gelöst, es werden 1,5 Mol Allylbromid zugesetzt und bei einer Temperatur von 55°C unter Rühren innerhalb von 2,5 Stunden erwärmt. Das erhaltene Produkt wird abfiltriert und mit Azeton gewaschen. Die Ausbeute am N-Allyl-2-pyridinaldoxim beträgt 77 Ma-%. Schm.p. = = 185 bis 186°C (aus Äthanol).

Gefunden, %: Br 32,83; 32,75; N 11,43, 11,55. $C_9H_{11}BrN_2O$.

Berechnet, %: Br 32,87; N 11,52.

Beispiel 3.

0,05 Mol $\alpha$-Pyridinaldoxim werden in 20 ml Dime-thylformamid gelöst, es werden 1,5 Mol Allylbromid zugesetzt und bei einer Temperatur von 40°C unter Rühren innerhalb von 8 Stunden erwärmt. Das erhaltene Produkt wird abfiltriert und mit Azeton durchgewaschen. Die Ausbeute am N-Allyl-2-pyridinaldoximbromid beträgt 50 Ma-%. Schm.p. = 185 bis 188°C (aus Äthanol).

Gefunden, %: Br 32,75; 32,86; N 11,47; 11,65. $C_9H_{11}BrN_2O$.

Berechnet, %: Br 32,87; N 11,52.

- 4 -

## Industrielle Verwendbarkeit

Das erhaltene N-Allyl-2-pyridinaldoximbromid stellt den Wirkstoff eines Antidots bei der Vergiftung mit phosphororganischen Verbindungen dar und findet Verwendung in der Medizin.

- 5 -

PATENTAPSPRÜCHE

1. Verfahren zur Herstellung von N-Allyl-2-pyridin-aldoximbromid durch die Umsetzung von $\alpha$-Pyridinaldoxim mit Allylbromid im Medium eines organischen Lösungsmittels mit darauffolgender Isolierung des Zielprodukts, dadurch g e k e n n z e i c h n e t , dass als organi-sches Lösungsmittel Dimethylformamid oder Dimethylsulf-oxyd ausgenutzt werden und der Prozess bei einer Temperatur von 40 bis 60°C durchgeführt wird.

2. Verfahren nach Anspruch 1, dadurch g e k e n n -z e i c h n e t , dass der Prozess bei einer Temperatur von 50 bis 60°C durchgeführt wird.

*S3400225.C*

# INTERNATIONAL SEARCH REPORT

*OO-70*

## I. CLASSIFICATION OF SUBJECT MATTER (if several classification symbols apply, indicate all) [6]

According to International Patent Classification (IPC) or to both National Classification and IPC

IPC[4]   C 07 D 213/53// A 61 K 31/44

## II. FIELDS SEARCHED

### Minimum Documentation Searched [7]

| Classification System | Classification Symbols |
|---|---|
| IPC[4] | C 07 D 213/53, A 61 K 31/44 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | GB, B, 1058820 (CIBA Limited) 15 February 1967 (15.02.67), see samples 1,2 | 1,2 |
| Y | The Journal of Organic Chemistry, volume 23, No. 5, May 1958, Americain Chemistry Society (USA), Edward J. Poziomek et al. "Pyridinium Aldoximes", pages 714-717 cited in the description | 1,2 |
| Y | E. Demlov et al. "Mezhfazny kataliz", 1987, Mir (Moscow), see pages 167,168 | 1,2 |
| Y | EP, A1, 0023378, (NEDERLANDSE ORGANISATIE VOOR TOEGEPAST-NATUURWETENSCHAPPELIJK ONDERZOEK TEN BEHOEVE VAN DE RIJKSVER-DEDIGING) 4 February 1981 (04.02.81) see sample 1 &, US, A, 4352810, 05.10.82 CA, A1, 1154771, 04.10.83 NL, A, 7905789, 28.01.81 | 1,2 |

---

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 30 December 1988 (30.12.88) | 24 February 1989 (24.02.89) |

| International Searching Authority | Signature of Authorized Officer |
|---|---|
| ISA/SU | |

Form PCT/ISA/210 (second sheet) (January 1985)